Europäisches Patentamt

(19) **European Patent Office**

Office européen des brevets

(11) Publication number: **0 009 577**
**A1**

(12) # EUROPEAN PATENT APPLICATION

(21) Application number: **79102932.5**

(22) Date of filing: **13.08.79**

(51) Int. Cl.³: **A 61 K 9/52**
**A 61 K 39/02**

(30) Priority: **18.08.78 US 935062**
**18.08.78 US 934812**

(43) Date of publication of application:
**16.04.80 Bulletin 80/8**

(84) Designated Contracting States:
**BE DE FR GB IT NL**

(71) Applicant: BURNS-BIOTEC LABARATORIES INC.
2000 Galloping Hill Road
Kenilworth New Jersey 07033(US)

(71) Applicant: Iowa State University Research Foundation,
Inc.,
315 Beardshear, Iowa State University Ames
Iowa 50010(US)

(72) Inventor: Harris, Delbert L.
4117 Phoenix Ames
Iowa 50010(US)

(72) Inventor: Goodnow, Robert A.
Rfd. 4 Box 45a
Omaha Nebraska 68137(US)

(74) Representative: Antony, Fritz, Dr. et al,
SCHERICO LTD.,P.O. Box 601 Winkelriedstrasse 35
CH-6002 Lucerne(CH)

(54) **An oral vaccine preparation and a method for increasing the resistance of swine to swine-dysentery infection.**

(57) This invention relates to preparations for increasing the resistance of swine to swine dysentery infection. The novel preparation of this invention comprises enteric-coated orally-administrable particulate material containing concentrated killed cells of a virulent isolate of *Treponema hyodysenteriae*. In the preferred method of use, the resistance of swine to dysentery infection is increased by administering a parenteral preparation prior to the oral preparation, which can comprise a series of oral doses following the parenteral dose.

EP 0 009 577 A1

Croydon Printing Company Ltd.

TITLE MODIFIED
see front page

Vaccine and Vaccination for Swine Dysentery

An anaerobic spirochete, Treponema hyodysenteriae, has been charcterized as the primary etiological agent in swine dysentery. Harris, D.L.; Glock, R.D.; Christensen, C.R.; and Kinyon, J.M.: Vet. Med./Small Animal Clin. 67:61 (1972); Taylor, D.J.; and Alexander T.J.L.: Brit. Vet. J. 127:108 (1971). But relatively little is known about the immunology of swine dysentery although resistance to reinfection can be demonstrated in convalescent pigs. In 1976, Glock et al reported that parenteral vaccination of pigs with killed cells of a virulent isolate of T. hyodysenteriae provided a significant degree of protection against subsequent intragastric challenge with live virulent T. hyodysenteriae. Glock, R.D., Schwartz, K.J., and Harris, D.L., Proceedings, International Pig Veterinary Society Congress, June 1976, Ames, Iowa. The vaccine was given in six intravenous injections at 6-day intervals. This was the first reported success in immunizing swine against swine dysentery infection. For field use, an oral vaccine would be more convenient to use. However, previous attempts to develop an oral vaccine have produced discouraging results.

Hudson et al found that oral dosing of an attenuated strain of _T. hyodysenteriae_ provided no protection against subsequent challenge. Hudson, M.J., Alexander, T.J.L., Lysons, R.J., Wellstead, P.D., Brit. Vet. J. (1974) 130:37. Subsequently, Hudson et al attempted to immunize pigs with live attenuated _T. hyodysenteriae_ using a combination of oral dosing and parenteral inoculation. Hudson, M.J., Alexander, T.J.L., Lysons, R.J., Prescott, J.F., Res. Vet. Science (1976) 21:366. Oral doses were administered on three consecutive days, and after an interval of several days, intraperitoneal vaccinations were administered, which were followed after several more days by intramuscular vaccinations. The overall results of these tests were summerized as follows: "Although vaccination appeared to enhance immunity to swine dysentery, half of the vaccinated pigs developed the disease. This level of protection would be unlikely to be of practical value in the field."

The present invention is based in part on the discovery that the resistance of swine to dysentery infection can be increased by orally administering enteric-coated particulate material containing concentrated killed cells of a virulent isolate of _Treponema hyodysenteriae._ The enteric coating is selected so that it will be resistant to dissolving in the swine stomach while dissolving in the swine intestines to release the killed cells for immunizing action. Preferably, the enteric coating is substantially insoluble in water at a pH below

0009577

5.0, while becoming slowly soluble in water at a pH of 5.8 to 6.2. Oral preparations prepared in accordance with the present invention are particularly useful for administration to young pigs which are subject to swine dysentery infection. For this purpose, the oral vaccine is preferably prepared in the form of enteric coated granules, which are mixed with a finely-divided feed material, and fed to pigs on the basis of a regimen which provides massive introduction of the killed cells into the colon area. Preferably, the immunizing feed is administered to the pigs at least once every 24 hours for a period of at least five days with each feeding providing at least 3 milligrams of the cells (dry basis) per animal.

The effectiveness of the oral vaccination can be still further improved by first administering a parenteral vaccine. The parenteral vaccine should also contain killed cells of a virulent isolate of <u>Treponema hyodysenteriae</u>. The parenteral vaccine appears to act synergistically with the delayed oral vaccine. Although the theoretical explanation for this action hs not been definitely established, it appears to involve a kind of antigenic sensitization. Such a phenomenon has been observed by Pierce et al with respect to cholera toxoid in rats. Pierce, N.F. and Gowens, J.L., "Cellular Kinetics of the Intestinal Immune Response to Cholera Toxoid in Rats", J. Exp. Med. (1975) 142:1550.

- 4 -

0009577

To achieve the maximum benefits of the present invention, it is desirable to wait at least five days after the parenteral vaccination before beginning a series of oral administration of enteric-coated pellets. For further sensitization of the animals, a second parenteral dose of the T. hyodysenteriae antigen may be given prior to or concurrently with the start of the oral series. In one embodiment, baby pigs are given one or two parenteral vaccinations prior to weaning, and the administration of the oral vaccine is started as soon as the pigs are receiving dry feed, which may be prior to weaning. The oral administration should be continued for several days, such as for at least five days, the oral vaccine being administered once every 24 hours in an orally effective amount.

The present invention can be practiced with any virulent isolate of T. hyodysenteriae. Attenuated or non-virulent isolates or strains are not desirable. A virulent isolate or strain is one which is capable of producing a typical swine dysentery infection. One suitable isolate has heretofore been identified in the literature as B204. See Kinyon, J.M., and Harris, D.L.: Vet. Rec. (1974): 95:219. The same publication also refers to an isolate identified as B234, which can also be used in practicing the present invention. However, type strain B78 (ATCC No. 27164) is not suitable, being non-virulent. Isolates B204 and B234 have been  deposited with the American Type Culture Collection; B204 being identified as ATCC No. 31212

and B234 as ATCC No. 31287. It should be understood that these isolates are but representatives of a class of virulent isolates or strains which can be employed.

The T. hyodysenteriae cells for preparation of the oral vaccine can be cultured using trypticase soy broth (TSB) with 10 % (v/v) fetal calf serum (FCS). For example, the inoculated broth can be incubated at 37-38$^{o}$C under an anaerobic atomosphere, such as 50:50 $H_2$:$CO_2$ or $CO_2$ alone. The gaseous atmosphere should be deoxygenated. For further details, see Kinyon, J.M., and Harris D.L.: Vet. Rec. (1974): 95-219.

After the fermentation has been completed, the cells can be recovered and concentrated by centrifugation or ultrafiltration to obtain a cell slurry for further processing. The cells are killed by a suitable procedure either in the fermenter or after recovery. Standard killing agents may be used such as formalin or merthiolate. For example, a killing-concentration of formalin, such as 0.2 % formalin (v/v), can be added to the fermenter or cell slurry. The slurry of killed cells is used to prepare the enteric-coated pellets.

To act as a filler or bulk stabilizer for desiccation and preparation of the particulate material, e.g. pellets and granules, standard filler substances may be added to the cell slurry such as sucrose, dextrose, lactose, etc. In general, the amount of filler-stabilizer to be added, e.g. by preparation of pellets, may range from about 10 to 50 parts by weight of filler per 100 parts of cells (dry basis). Prior to the addition of the filler, the cell concentrate preferably contains in excess of 3.0 milligrams of cells (dry basis) per milliliter of slurry. For example, the cell concentrates may contain from about 4 to 7 milligrams of cells (dry basis) per milliliter of cell slurry. The particular concentration is not critical, since most of the residual water of the slurry is removed by a suitable drying procedure in preparing the pellets.

For example, a 1:10 dilution of sterile 50 % non-fat powdered milk plus 50 % $H_2O$ solution to act as filler-stabilizer was added to the concentrated slurry (4.0 mg/ml dry weight antigen). This material was dried by a biological drying procedure such as freeze-drying.

The cell slurry containing the added filler may be dried by a biological drying procedure such as freeze-drying. Preferably, the drying is carried out at a relatively low temperature, such as not over 40$^O$C. The dried material is then pulverized to a finely-divided condition for preparing tablets or granules. If desired, a binder material such as starch may be added at this point, although the filler may provide adequate binding. Tablets may then be prepared by tableting in a standard tablet press, either manual or automatic types.

- 7 -

0009577

Alternatively, the dried cells may be formed into granules by a known granulation procedure. For example, the cell concentration in the form of a liquid slurry produced by ultrafiltration is mixed with sucrose and cellulose, and kneaded to a doughy consistency. The cell dough is then extruded in the form of noodles or ribbons, which are broken up and formed into granules by a granulation apparatus. The granule size is not critical, but desirably is of a small size, such as below 20 mesh (U.S. Standard Screen). The granules are dried in an oven at a relatively low temperature, such as 37-40°C until most of the moisture has been removed. The final moisutre content is not critical, and desirably may range from about 1 to 3 % water by weight.

Orally-administrable particulate material, which may comprise pellets, tablets or granules as described above, are provided with an enteric coating for the purpose of the present invention. The material used to provide the enteric coating should be selected so that it is resistant to dissolving in the swine stomach, while dissolving in the swine intestines. The differences in pH between the stomach and intestines can be used to control the dissolving of the enteric coating. While the pH conditions of the stomach and intestines of swine vary with diet and time of day, in general, the pH of a swine stomach and of the gastric fluid is below 5.0, such as 4.0 to 4.5. The pH of the small gut may range from about 5.5 in the upper

0009577

small gut to about 5.9 to 6.1 in the middle and lower small gut. Similarly, the pH of the large bowel and colon may range from about 5.8 to 6.1. Preferably, therefore, the enteric coatings used in the present invention are substantially insoluble in water at a pH below 5.0, while being slowly soluble in water at a pH of 5.8 to 6.2. With this mechanism, the coatings are not exposed to the pH conditions of the mouth long enough to be dissolved and the coatings can remain sufficiently intact in the stomach to protect most of the vaccine from destruction by the gastric juice. During transit through the small bowel, the coatings on the granules are gradually dissolved, and are completely dissolved or disintegrated at least by the time the vaccine reaches the colon. The killed cells are therefore released in the swine intestines for immunizing action in the swine colon.

When the swine are being fed continuously, such as with feeder pigs, the pH of the food mass in the stomach may vary from about 4.0 near the stomach wall to 5.7 to 6.1 at the center of the food mass. The average residence time of the food in the stomach may range from 1 to 2 hours. Preferably, therefore, the coatings on the vaccine granules should require at least one hour to dissolve in water at pH 6.0 and 37$^{\circ}$C (the standard temperature).

Any enteric coatings which meet the foregoing pH conditions can be utilized in practicing the present invention. One enteric coating material which can be used is cellulose acetate phthalate. As is well known in the art, cellulose acetate phthalate may be plasticized with diethyl or dibutyl phthalate so that the coating is more resistant to cracking. For application, the enteric coating material may be dissolved in a suitable volatile organic solvent, and the enteric coat may be built up in a series of applications to assure that the coating will be complete and relatively uniform. One known procedure of this kind is referred to as the "Open-Pan Ladle Coating Process". For example, 30 to 40 parts by weight of cellulose acetate phthalate together with 8 to 10 parts of diethyl phthalate may be dissolved in 250 to 300 parts by weight of acetone to form a coating solution. Suitable enteric coating resins may also be used, such as acrylic resins prepared for use as enteric coatings. One such product is sold under the trademark "Eudragit L" by Röhm Pharma GmbH, Darmstadt, West Germany. The release pH of Eudragit L can be increased where desired by mixing it with Eudragit S. The manufacturer describes Eudragit L as soluble in intestinal juice from pH 6.0 and Eudragit S as soluble from pH 7.0. These enteric coating materials are supplied in granular form containing 10 % moisture; specified as Eudragit L90 and Eudragit S90 to indicate the 90 % active content. Eudragit L or mixtures of L and S are soluble in alcohols and acetone which may be used for applying the coating. The coating materials may be plasticized with various plasticizers, such as polyethylene glycol, dibutylphthalate, triacetin, or castor oil. Satisfactory coating results, however, can be obtained using the Eudragit material without plasticizer addition.

The Wurster Coating Process can also be used to apply the enteric coatings. This process is described in United States Patents 3,241,520 and 3,253,944. It is carried out as a commercially available service by Coating Place, Inc., Verone, Wisconsin.

Where the swine are being fed continuously, a preferred coating contains from 5 to 20 parts by weight of Eudragit S in admixture with from 95 to 80 parts of Eudragit L. For example, 15 parts S with 85 parts L increases the release pH so that at least 75 % and up to 90 % of the coated granules pass through the stomach with the protective coatings substantially undissolved. There is complete release in the intestines because of the longer residence time (8 to 24 hours).

The enteric-coated oral vaccine of the present invention is preferably in the form of granules which can be readily mixed with swine feed material for administration to the animals. For example, such granules may range from about -20 mesh to +100 mesh (U.S. Standard Screen). The granules are mixed with a finely-divided feed material such as a ground feed used for baby pigs after weaning. Any swine or pig feed material can be used, such as a basal ration containing ground corn, rolled oats, soybean meal, minerals, and vitamins. The coated granules may also be mixed with vitamin-mineral fortified premixes which are later combined with the other feed ingredients by the pig raisers. Such pre-mix may also contain high protein feed materials such as soybean meal.

While the enteric-coated vaccine particulate material of the present invention and the oral method of immunization may be applied to adult swine, such as breeding sows, an important use is with growing pigs. For example, the method may be applied to feeder pigs as soon as they are receiving solid feed, either shortly before or following weaning. For example, the vaccine administration may be started at the age of about 3 to 8 weeks. The method may also be applied to older pigs during their growth period prior to marketing. The pigs raised under field conditions are highly subject to swine dysentery infection with consequent economic loss due to lowering of the rate of weight gain and the feed efficiency. By increasing the resistance of the pigs to swine dysentery infection, optimum rates of weight gain may be maintained.

In practicing the present invention, it is desirable to administer a plurality of doses of the enteric-coated oral vaccine, such as from 3 to 15 doses. Preferably, such dose should provide at least 3 milligrams of the killed cells of _T. hyodysenteriae_ (dry basis) per animal; the doses being administered daily (once every 24 hours), such as by admixture of the enteric-coated granules with a feed material. This dosing may be continued for a period of at least 5 days, such as from 5 to 15 days. In a preferred embodiment, the oral doses contain at least 4 mg of the killed cells (dry basis) per dose, such as doses in the range of 4 to 6 mg. Since 1 mg of the cells (dry basis) contains about $2.5 \times 10^9$ cells, a dose of 5 mg will provide approximately $1.2 \times 10^{10}$ cells. Therefore, in accordance with the present invention, massive quantities of the killed cells are delivered to the swine colon for immunizing action therein.

In applying tablet and granule coatings, a dye may be included as a colorant for the coating. This permits the coating to be more readily inspected for thickness and uniformity, and makes it easier to detect imperfections in the coatings. In practicing the present invention, it is desirable to use a dye in the enteric coatings of the present invention, although it is not essential with respect to the desired immunizing action. Suitable dyes include Lake Blue No. 2 and crystalline violet dye.

In a preferred method of using the oral vaccine of this invention, a sequence of parenteral and oral administrations are

- 13 -

0009577

carried out before the swine have contracted the infection. There is first parenterally administered to the swine an injectable cell concentrate containing a virulent isolate of killed cells of Treponema hyodysenteriae. At least one injection is given per animal and each injection should contain at least 2 milligrams (dry basis) of the antigenic cells. The parenteral injection may be repeated, such as a total of 2 to 3 parenteral injections, but it does not appear that more than 2 injections are needed. In one embodiment, the first parenteral injection is given, and after an interval of at least 4 days, a second parenteral injection is administered. The second injection may be prior to or concurrently with the starting of the administration of the oral vaccine. For example, weaned pigs may be given a parenteral injection, and after a delay of 5 to 8 days, a second parenteral injection is given concurrently with the starting of the oral vaccination series. In a variation of this procedure, baby pigs prior to weaning are given at least one parenteral vaccination, and are given the second parenteral vaccination shortly before weaning, and either before or after weaning, the oral vaccination series is started. Such variations come within the scope of the present invention, the essential feature of the method of the present invention being that the parenteral vaccination precedes by several days the oral series. The desirability of this sequence was not recognized by prior art workers, testing oral vaccination for swine dysentery. Hudson et al administered the oral doses first, and after delay of several days, the parenteral vaccinations were administered. See Hudson et al <u>Res. Vet. Science</u> (1976) 21:366.

In accordance with the present invention, not less than 5 days after the animals have received the first of the parenteral injections, a series of oral administrations is begun of enteric-coated pellets containing killed cells of a virulent isolate of <u>Treponema hyodysenteriae</u>. These oral administrations are preferably given once every 24 hours for a period of at least 5 days. Each oral dose should comprise at least 3 milligrams (dry basis) of the <u>T. hyodysenteriae</u>. In one preferred procedure, the oral administrations provide at least 4 milligrams (dry basis) of the antigenic cells per animal, and are continued for at least 8 days at the rate of one oral administration per animal per 24 hours. While the method of this invention can be applied to adult swine, such as breeding sows, it has particular importance for use with growing pigs. As a matter of convenience, the oral administrations are preferably not started until the pigs are receiving dry feed, which may be prior to weaning. The method is particularly applicable to young growing pigs which are referred to as feeder pigs. The parenteral vaccinations, which can be conveniently carried out by intramuscular or subcutaneous injection, can be given before the pigs are weaned, or both before and after weaning.

This invention is further illustrated by the following examples.

## EXAMPLE I

Organism: The microorganism, <u>Treponema hyodysenteriae</u>, Strain B204 (ATCC No. 31212) was grown in Trypticase Soy Broth containing 5-10 % sterile Fetal Calf Serum (GIBCO Laboratories, Grand Island, N.Y.) in test tubes, 500 cc Erlenmeyer flask, 4-liter glass jugs and in a 25-liter pilot New Brunswick fermentor. The growth culture was maintained until log phase growth ceased. The culture was inactivated by addition of formalin to 0.2 % concentration. 50:50 $H_2$:$CO_2$ or no gas was used to maintain the gaseous atmosphere in the fermentor during the growth period. After the fermentation has been completed, the cells were concentrated in liquid mass by ultrafiltration.

The cell culture was forced by positive air pressure through a 100,000 molecular weight size membrane. The cell slurry was concentrated to a final concentration of 4.0 milligrams dry weight measurement per ml of culture. Ten percent (10 %) by volume sterile milk stabilizer (50 % Non-fat dry milk powder in 50 % $H_2O$) was added to the antigen <u>T. hyodysenteriae</u> slurry. E.g. 750 ml culture at 4 milligrams antigen/ml or 3.0 grams of antigen added to 75 ml milk stabilizer containing 37.5 grams dry powdered milk and 37.5 ml $H_2O$. The antigen

milk stabilizer mixture was freeze-dried in a commercial freeze drier for 18 hours. The temperature was never allowed above 37°C during the drying of the mixture. 66.20 grams of dried material was recovered from the drying process.

Preparation of enteric-coated pellets: Ten percent (10 %) by weight dry soluble starch was added to the dried material giving the following components:

    3.0  grams active antigen T. hyodysenteriae
              (killed cells-dry basis)

    0.3  grams powdered milk stabilizer

    0.33 grams soluble starch binder

0.5 Gram mixture (3 mg dry basis active T. hyodysenteriae antigen) of the above mixture was placed in a hand-operated pellet press. A 3/8" punch and die set was used to give a table of approximately 3/8" thickness by 1/2" in diameter.

Each tablet was coated with an enteric coating which upon contact in a chemical environment pH 6 or greater, will degrade and release the antigenic material into the colonic lumen. The coating is not soluble at the stomach pH.

Enteric Delivery System:

1. Enteric Coating Solution (36.0 grams of cellulose acetate phthalate, 9.0 grams of diethyl phthalate, and 0.2 grams of crystalline violet dye) was diluted into 255 grams of reagent grade acetone. (For a description of a similar procedure, see C.J. Malm, J. Amer. Phar.
Assoc., Science Edition, Vol. 40, p. 520, 1951.)

2. Enteric Coat Application - Each tablet was air blasted
to remove dust and was submerged under the surface of
the coating solution at least twenty times, with complete air drying of tablets between coatings.

3. Wax Coating - The enteric coating was further protected
from $H_2O$ uptake by application of a white wax ("Be Square"
195, Petrolite Corporation, Bareco Division, Tulsa, Oklahoma). This was liquified in the GI tract, and did not
inhibit the antigen release in the colon. Ten (10) grams
of "Be Square" 195 was beads were dissolved in 10 ml of
acetone.

Each enteric coated tablet was submerged at least twice
in the liquid wax solution. The wax coat was allowed to
dry prior to tablet bulking and storage. Stearic acid or
other fatty acid or fatty acid triglyceride can also be
used as an auxilliary coating to further protect the
vaccine in the stomach. The fatty acids and triglycerides will be digested off the granules in the small gut.

Parenteral Vaccines:

The test also included administration of parenteral vaccine
prepared as follows: The concentrated liquid slurry described above (4.0 mg T. hyodysenteriae cells dry basis per ml) was diluted from 4.0 mg/ml to 1.0 mg/ml with a 20 % solution of sterile aluminum hydroxide adjuvant. The resulting parenteral vaccine contained about $2.5 \times 10^9$ killed cells per milliliter.

Materials and Methods

Experimental Animals--Sixteen (16) pigs from a herd with no history of swine dysentery were placed in swine testing facilities at approximately five weeks of age and fed a 16 % protein grown ration containing no drugs.

Preparation of Vaccines--An orally delivered enteric release vaccine and a parenteral vaccine containing Treponema hyodysenteriae Isolate B204 (ATCC No. 31212) with aluminum hydroxide as adjuvant was prepared.

Preparation of Inoculum--Cultures of Treponema hyodysenteriae (Isolate B204) were grown approximately 24 hours in aerobically prepared trypticase soy broth containing 10 % fetal calf serum under deoxygenated $H_2:CO_2$ at 37°C. Seventy-five (75) ml of whole culture containing approximately $5 \times 10^8$ organisms per ml was administered to each pig via stomach tube following a 48 hour starvation period. The isolate of T. hyodysenteriae had not been passaged more than eight (8) times in vitro.

0009577

Experimental Design--The sixteen (16) pigs were randomly assigned to individual pens. The pigs were immunized as follows:

| GROUP | NO. OF PIGS | PARENTERAL VACCINE SUBCUTANEOUS* | INTRAPERITONEAL* | ORAL VACCINE* |
|-------|-------------|----------------------------------|------------------|---------------|
| I | 4 | - | + | + |
| II | 4 | + | - | + |
| III | 8 | - | - | - |

* + = administered;  - = not administered.

On day 0, the pigs in Group I were injected with 5 ml/pig of the parenteral vaccine. Pigs in Group II were injected with a similar amount/pig by subcutaneous route. On day 14, a 5 ml/pig booster vaccination was given by respective routes. On day 19, each pig in Groups I and II were given one (1) oral tablet per day through the 29th day. On day 30, all pigs were weighed. Feed was withheld on days 30 and 31. On day 32, rectal swabs were collected and all pigs were challenged with T. hyodysenteriae as described above. Clinical evaluation of response to challenge was recorded for each pig on a twice daily basis for 40 days post inoculation. On day 40 post-inoculation, each pig was weighed.

Evaluation of Response to Challenge--Each pig was observed twice daily and 3 clinical parameters were scored on a scale of 1 to 3. The results are shown in Table A.

TABLE A

| Clinical Response | Group | | |
| --- | --- | --- | --- |
| | I | II | III |
| Diarrhea: | N=4[a] | N=4 | N=8 |
|     Day of Onset[b]/Post. Inocul. | 16 | 5.2 | 13.6 |
|     Days Duration | 4 | 8.2 | 11.8 |
|     No. Affected | 4 | 4 | 6 |
| Dysentery: | | | |
|     Day of Onset/Post Inocul. | 25.7 | 24.2 | 17.6 |
|     Days Duration | 3.2 | 2.5 | 5 |
|     No. Affected | 2 | 2 | 5 |

[a] N equals number of pigs per group

[b] Study terminated at 40 days. Calculations are based on a value of 40 assigned to each pig which remained normal.

General Condition:  1 = Normal 2 = Gaunt, mildly inactive 3 = Emaciated, moribund

Feces Consistency:  1 = Normal, firm 2 = Soft, not formed 3 = Liquid

Feces Composition:  1 = Normal 2 = Increased mucus 3 = Large amount of blood present

Observation of T. hyodysenteriae Like Organisms--Rectal swabs were collected. A drop of each sample was reviewed under a dark field microscope observation. There was significant increase in the numbers of T. hyodysenteriae like organisms in clinically affected pigs which was considered evidence of an ongoing swine dysenteriae infection.

Weight Gain--The average weight gain during the period from day of challenge inoculation to the day of study termination was compared between vaccinated and non-vaccinated groups. The results are shown in Table B.

### TABLE B

| Days | Group | | |
|------|-------|----|-----|
| | I | II | III |
| Post Test Initiation | IP-Oral | SC-Oral | Controls |
| 0 | 26.0 | 27.6 | 31.1 |
| 40 | 103.0 | 110.2 | 94.0 |
| Total Average Gain per pig | 77.0 | 82.6 | 62.9 |

### EXAMPLE II

Enteric coated granules can be prepared by concentrating fermentor grown Treponema hyodysenteriae culture to about 64 mg dry weight/ml. Using such a concentrated antigen slurry it is combined with the other ingredients in the following .proportions:

> 1500 cc antigen slurry
>
> 11.5 kilo sucrose
>
> 3.5 kilo microcrystalline cellulose
>
> 0.2 % dry weight Lake blue No. 2 dye
>
> $H_2O$ added as needed for obtaining proper texture

Once this mixture is partially mixed the moistened mass of material is run through a commercial extruder at least three times to provide a uniform mix of antigen to carrier. The cylindrical pieces are then shaped into uniform beads in a manumerizer. The bead preparation is dried at least 1 - 8 hours leaving 1 - 3 % moisture content. An enteric coating is then applied to the beads by either the Wurster or an open-pan, ladle type coating process, as previously identified. A preferred coating is a mixture of 85 parts by weight of Eudragit L 90 with 15 parts Eudragit S 90, using acetone or ethanol as the carrier solvent for applying the coating. (Eudragit L 90 and S 90 are sold by Röhm Pharma GmbH, Darmstadt, West Germany.)

The enterically coated beads prepared as described will contain approximately 10 % containing immunizing antigen (dry basis) can be administered in the following manner:

A. Remove all feed from swine (viz. weaned baby pigs) to be immunized 24 hours prior to treatment.

B. Mix the enteric coated granules with 10 - 20 % of the daily required feed in a 1:1 ratio.

C. Each pig would receive an average of 6 - 10 mg immunizing antigen/day, which determines the average amount of feed to be given to a group of pigs.

D. Feed untreated feed free choice during part of the day, but withhold feed overnite.

E. Repeat this feeding and withholding procedure for 5 - 10 days.

## EXAMPLE III

Antigenic vitamin-mineral premixes can be prepared by mixing the immunizing granules prepared as described in Example II with standard pig feed fortification premixes. For example, the granules containing 10 % of the T. hyodysenteriae cells (dry basis) are mixed with a standard premix in the amount 100 grams of granules per 7.5 pounds (3.375 kilograms) of a vitamin-mineral premix. The premix is then incorporated in pig feed in the amount of 7.5 pounds (3.375 kilograms) per 2,000 lbs. (900 kilograms).

0009577

## CLAIMS

1. An oral preparation for increasing the resistance of swine to swine dysentery infection, comprising enteric-coated orally-administrable particulate material containing concentrated killed cells of a virulent isolate of <u>Treponema hyodysenteriae</u>, said enteric coating being resistant to dissolving in the swine stomach while dissolving in the swine intestines to release said cells for immunizing action.

2. The oral preparation of claim 1, further characterized by said killed cells of <u>Treponema hyodysenteriae</u> being prepared from isolate B204 (ATCC No. 31212) or B234 (ATCC No. 31287).

3. The oral preparation of claim 1 or claim 2, further characterized by said enteric coating being substantially insoluble in water at a pH below 5.0 while being slowly soluble at a pH of 5.8 to 6.2, the coatings on said pellets requiring at least one hour to dissolve in water at $37^{O}C$ and pH 6.0.

4. The oral preparation of any one of claims 1 to 3, characterized by also containing vitamins and minerals, thereby forming an antigenic premix for administration to growing pigs.

5. The method of increasing the resistance of swine to dysentery infection, characterized by a sequence of parenteral and oral administrations which are carried out before the swine contract the infection, as follows:

(a) first parenterally administering to the swine an injectable cell concentrate containing killed cells of a virulent isolate of Treponema hyodysenteriae, at least one injection being given per animal and each injection containing at least 2 milligrams (dry basis) of said cells; and

(b) not less than 5 days after said animals have received the first of said parenteral injections, beginning a series of oral administrations of enteric-coated pellets containing concentrated killed cells of a virulent isolate of Treponema hyodysenteriae, said oral administrations being given at least once every 24 hours for a period of at least 5 days, and each of said oral administrations providing at least 3 milligrams (dry basis) of said cells.

6. The method of claim 5, further characterized by said killed cells of Treponema hyodysenteriae being prepared from isolate B204 (ATCC No. 31212) or B234 (ATCC No. 31287).

7. The method of claim 5 or claim 6, further characterized by said parenteral injections comprising 2 injections per animal with an interval of at least 4 days between the days on which the injections are given, and each parenteral injection providing at least 3 milligrams (dry basis) of said cells of Treponema hyodysenteriae.

8. The method of claim 5 or claim 6, further characterized by each of said oral administrations providing at least 4 milligrams (dry basis) of said cells per animal and being continued for at least 8 days at the rate of one oral administration per animal per 24 hours.

9. The method of claim 5 or claim 6, further characterized in that swine are growing pigs, and said oral administrations are started after the pigs have been weaned.

European Patent Office

**PARTIAL EUROPEAN SEARCH REPORT**
which under Rule 45 of the European Patent Convention shall be considered, for the purposes of subsequent proceedings, as the European search report

0009577
Application number

EP 79 10 2932

## DOCUMENTS CONSIDERED TO BE RELEVANT

| Category | Citation of document with indication, where appropriate, of relevant passages | Relevant to claim |
|---|---|---|
| | <u>US - A - 4 100 272</u> (R.D. GLOCK et al.)<br>  * Claims *<br><br>-- | 1,2,5 |
| | <u>US - A - 3 823 228</u> (D.H. FERRIS et al.)<br>  * Claims 1-10 *<br>& FR - A - 2 197 576<br>& NL - A - 73 12032<br>& GB - A - 1 440 214<br><br>-- | 1,3 |
| | <u>FR - A - 1 560 264</u> (AMERICAN HOME PRODUCTS)<br>  * Abstract *<br><br>-- | 1,3 |
| | <u>FR - A - 2 123 421</u> (H. STICKL)<br>  * Claims *<br><br>-- | 1,3 |
| D | RESEARCH IN VETERINARY SCIENCE, vol. 21, no. 3, 1976          ./. | 5 |

**CLASSIFICATION OF THE APPLICATION (Int. Cl.³)**

A 61 K  9/52
          39/02

**TECHNICAL FIELDS SEARCHED (Int. Cl.³)**

A 61 K  9/52·
          39/00
          39/02

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:        1-4
Claims searched incompletely:
Claims not searched:               5-9  Method for treatment of
Reason for the limitation of the search:     the human or animal body
by surgery or therapy (See article 52(4) of the European Patent Convention).

| Place of search | Date of completion of the search | Examiner |
|---|---|---|
| The Hague | 14-12-1979 | WILLEKENS |

EPO Form 1505.1  06.78

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | pages 366-367 M.J. HUDSON et al.: "Swine dysentery: protection of pigs by oral and parenteral immunisation with attenuated Treponema hyodysenteriae" * Pages 366-367 * -- | | |
| P,X | US - A - 4 152 413 (R.A. GOODNOW) * Claims * -- | 1-9 | |
| P,X | US - A - 4 152 414 (D.L. HARRIS et al.) * Claims; column 8, lines 1-27 * -- | 1-9 | TECHNICAL FIELDS SEARCHED (Int. Cl.³) |
| P,X | US - A - 4 152 415 (D.L. HARRIS et al.) * Claims * ---- | 1-9 | |